(19) **Europäisches Patentamt**
**European Patent Office**
**Office européen des brevets**

(11) **EP 1 935 334 B1**

(12) **EUROPEAN PATENT SPECIFICATION**

(45) Date of publication and mention
of the grant of the patent:
**01.07.2015 Bulletin 2015/27**

(51) Int Cl.:
***A61B 5/028*** (2006.01)

(21) Application number: **06127135.9**

(22) Date of filing: **22.12.2006**

(54) **Patient monitoring apparatus for determining a parameter representing an intrathoracic volume compartment of a patient**

Patientenüberwachungsgerät zur Bestimmung eines Parameters einer intrathorakalen Volumenkammer eines Patienten

Appareil de surveillance d'un patient pour déterminer un paramètre représentant un compartiment du volume intrathoracique d'un patient

(84) Designated Contracting States:
**DE ES FR GB IT**

(43) Date of publication of application:
**25.06.2008 Bulletin 2008/26**

(73) Proprietor: **Pulsion Medical Systems AG**
**81829 München (DE)**

(72) Inventors:
• **Thalmeier, Thomas**
**84405 Dorfen (DE)**
• **Dr. Goedje, Oliver**
**82031, Grünwald (DE)**
• **Dr. Scheier, Jörg**
**80634, München (DE)**

(74) Representative: **Ascherl, Andreas et al**
**KEHL, ASCHERL, LIEBHOFF & ETTMAYR**
**Patentanwälte - Partnerschaft**
**Emil-Riedel-Strasse 18**
**80538 München (DE)**

(56) References cited:
**US-A- 5 383 468       US-A- 5 526 817**
**US-A1- 2005 267 378**

• **JOHN D. CURRENT: "A Linear Equation For Estimating The Body Surface Area In Infants And Children" THE INTERNET JOURNAL OF ANESTHESIOLOGY, [Online] vol. 2, no. 2, 1998, pages 1-4, XP002433837 ISSN: 1092-406X Retrieved from the Internet: URL:http://www.ispub.com/ostia/index.php?xmlPrinter=true&xmlFilePath=journals/ija/vol2n2/bsa.xml> [retrieved on 2007-05-15]**
• **SAKKA S G ET AL: "Assessment of cardiac preload and extravascular lung water by single transpulmonary thermodilution." INTENSIVE CARE MEDICINE FEB 2000, vol. 26, no. 2, February 2000 (2000-02), pages 180-187, XP002433835 ISSN: 0342-4642**
• **LIVINGSTON EDWARD H ET AL: "Body surface area prediction in normal-weight and obese patients" AMERICAN JOURNAL OF PHYSIOLOGY, vol. 281, no. 3 Part 1, September 2001 (2001-09), pages E586-E591, XP002433836 ISSN: 0002-9513**
• **DAVID M. BERKOWITZ ET AL: "Accurate characterization of extravascular lung water in acute respiratory distress syndrome*", CRITICAL CARE MEDICINE, vol. 36, no. 6, 1 June 2008 (2008-06-01), pages 1803-1809, XP055159206, ISSN: 0090-3493, DOI: 10.1097/CCM.0b013e3181743eeb**

**(Cont. next page)**

- H FUKUSHIMA ET AL: "Indexing extravascular lung water to predicted body weight increases the correlation with lung injury score in patients with acute lung injury/acute respiratory distress syndrome: a prospective, multicenter study conducted in a Japanese population", CRITICAL CARE, vol. 15, no. Suppl 1, 1 January 2011 (2011-01-01), page P142, XP055159204, ISSN: 1364-8535, DOI: 10.1186/cc9562
- CHARLES R. PHILLIPS ET AL: "Extravascular lung water in sepsis-associated acute respiratory distress syndrome: Indexing with predicted body weight improves correlation with severity of illness and survival*", CRITICAL CARE MEDICINE, vol. 36, no. 1, 1 January 2008 (2008-01-01), pages 69-73, XP055159207, ISSN: 0090-3493, DOI: 10.1097/01.CCM. 0000295314.01232.BE
- O.J. LINARES-PERDOMO ET AL: 'Variations in Tidal Volume During Mechanical Ventilation Based On Different Predicted Body Weigh Equations. A Retrospective Study' 20 May 2013, AMERICAN THORACIC SOCIETY INTERNATIONAL CONFERENCE ABSTRACTS, XP055159244

**Description**

The present invention relates to patient monitoring apparatus, in particular to patient monitoring apparatus for determining a parameter representing an intrathoracic volume compartment of a monitored patient.

Patient monitoring apparatus are commonly used in modern day hospitals for monitoring the condition of the circulatory system of critically ill patients. As is well known to the person skilled in the art, patient monitoring apparatus may function according to one of a variety of measurement and evaluation principles, such as (right heart or transpulmonary) thermodilution, dye dilution, pulse contour analysis, or may combine two or more of these measurement and evaluation principles. Applying suitable methods according to these principles yields a variety of parameters which enable the physician in charge to judge the present condition of the patient and to take appropriate counter measures, if the condition should worsen. Particularly important among these parameters are intrathoracic volume compartments (often also referred to simply as intrathoracic volumes) such as global enddiastolic volume GEDV, extravascular lung water EVLW, pulmonary blood volume PBV and intrathoracic blood volume ITBV. For example, GEDV is used to assess the filling state of a patient, and EVLW is an important parameter for observing the development of a pulmonary oedema.

Patient monitoring apparatus for determining one or more of the above parameters are described inter alia in US 5,526,817 , US 6,394,961 , US 6,537,230 and US 6,736,782.

In the patent application US 5 383 468 A a cardiac output and right ventricular ejection fraction computer is disclosed which comprises a catheter, having an indicator injection port which is located at the right ventricular, inserted into an Patent Application No. 06127135.9-1526 intravascular of a patient and retained therein; thermal detector device, mounted on a distal portion of the catheter, for detecting the temperature of blood ejected from the right ventricle; signal processor device for calculating the right ventricular ejection fraction from a thermodilution curve obtained by detecting variation of the blood temperature measured by the thermal detector device when a indicator is injected into the right ventricular through the catheter; data input device for inputting data to the signal processor device.

In "Assessment of cardiac preload and extravascular lung water by single transpulmonary thermodilution" (Intensive Care Medicine Feb 2000, vol. 26, no. 2, February 2000 (2000-02), pages 180 - 187, XP002433835, ISSN: 0342 - 4624) by Sakka S. G. et al. a form of calculation of normalised parameters representing the Intrathoracic volume compartment (ITBVI, EVLWI) for intraindividual comparison is disclosed.

According to Livingstone, Edward H. et al. the "Body surface area prediction in normal-weight and obese patients" (American Journal of Physiology, vol. 281, no. 3 part 1, September 2001 (2001-09), pages E586 - E591, XP002433836, ISSN: 0002 - 9513) is described, wherein a function to relate body surface area to body weight is explained.

In "A linear Equation for estimating the body surface area in infants and children" (The Internet Journal of Anesthesiology, vol. 2, no. 2, 1998 (2001-09), pages 1 - 4, XP002433837, ISSN: 1092 - 406X) by John D. Current an Examination of published human body surface area (BSA) data, disclosing a linear relationship between BSA and weight in infants and children weighing between 3 and 30 kg. Application of linear regression analysis to published data results in a formula relating BSA in square centimeters and weight in grams.

Before drawing medical conclusions from the value of a specific intrathoracic volume compartment it is often necessary to take into consideration the body dimensions of a particular patient. For example, the identical value of extravascular lung water may indicate the development.of an edema when determined for a small patient, whereas it may suggest a noncritical situation when determined for a large patient. Therefore, extravascular lung water is often normalised (or indexed) by dividing it by the patient's actual weight. However, it has been found that considerable deviations may occurr in the case of very corpulent or very lean patients. Such deviations can cause errors in a diagnosis based on the normalised (or indexed) value.

In view of the above, it is an object of the present invention to technically improve the provision of intrathoracic volume data determined by a patient monitoring apparatus as a starting point for further medical diagnosis in order to provide a basis for facilitated (and thus more reliable) diagnostic interpretation.

According to the present invention, this object is accomplished as defined in the independent claims.

Advantageous embodiments of may be implemented according to any of the dependent claims.

According to the present invention, a parameter representing an intrathoracic volume compartment is thus normalised on the basis of input biometric data, such as body height, using an algorithm selected from a plurality of algorithms depending on a category a patient is allocated to. The categorizing information, on which this allocation is based upon, may be taken from a set of discrete data determining a category directly (such as gender, adult patient/ pediatric patient, underweight/overweight/neither underweight nor overweight) or from a continuous data range such as patient's age, waist circumference or body height. Input biometric information may thus also be used as categorizing information.

For ease of reading only, parameters representing an intrathoracic volume compartment of a patient will also be referred to as intrathoracic volume parameters hereinafter.

The selection of an algorithm, according to the present invention, can be implemented in many different ways, such as

- selecting, from a plurality of prestored program modules or sub routines, a program module or sub routine, respectively, to be carried out, or
- substituting coefficients and/or constants in a calculation formula or correction function, or the like.

In fact, any branch in a course of calculation implemented for normalising an intrathoracic volume parameter, wherein input categorizing data determines which calculation path is to be followed, is to be considered a selection of algorithms according to the present invention.

The present invention makes it thus possible to reduce or even eliminate errors induced by considering intrathoracic volume parameters without taking into account a patient's actual build. In fact, by applying a patient category dependent normalisation algorithm, the present invention automatically includes the consideration of general differences that are to be expected between two individuals belonging to a different category (e.g. a different age group or gender), even if they share the same biometric datum (such as body height), on which normalisation is based.

If, for example, extra vascular lung water of an overweight patient is normalised using his "predicted weight" or "ideal weight" instead of his actual body weight, values will be correctly perceived to be dangerous with regard to development of an edema, which otherwise may have been erroneously perceived to be normal.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise. It goes without saying that the present invention may be advantageously implemented for determining not just one but a plurality of parameters respectively representing various intrathoracic volume compartments of a monitored patient some or all of which can be normalized in the inventive manner.

The accompanying drawings, which are schematic illustrations, serve for a better understanding of the features of the present invention.

Fig. 1    schematically illustrates an example of a patient monitoring apparatus according to the present invention wherein determination of parameters representing intrathoracic volume compartments is based on transpulmonary thermodilution measurements.

Fig. 2    illustrates an examplary procedure of selecting a predicted weight determining algorithm in accordance with the present invention.

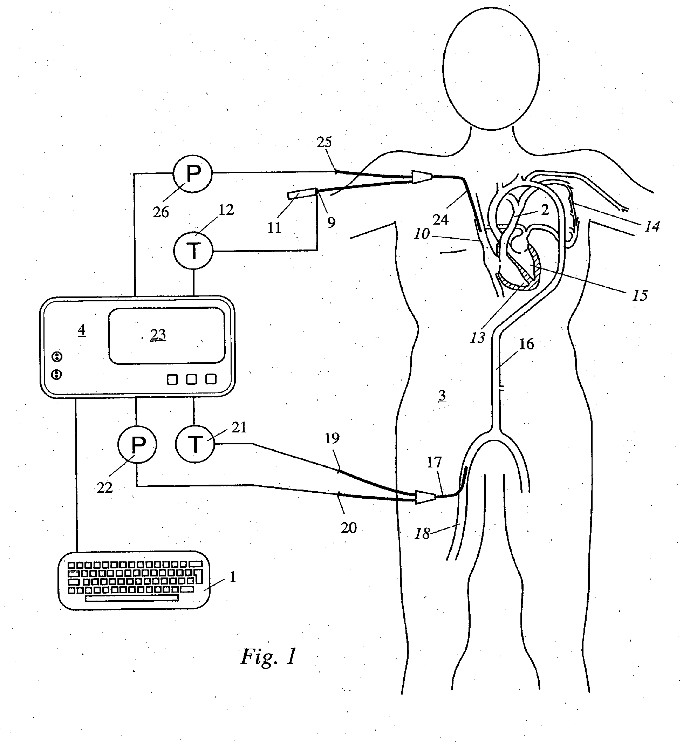

In addition to hardware and software for transpulmonary thermodilution measurements, the patient monitoring apparatus depicted in fig. 1 is further equipped with hardware and software for performing pulse contour analysis. Generally, the present invention is not limited to such a setup, but may be implemented using hardware and software for determining parameters representing intrathoracic volume compartments on the basis of dye dilution, combined thermodilution and dye dilution, or other known techniques.

The apparatus comprises a central venous catheter 24 extending into a central vein 10 of the patient 3. The central venous catheter 24 comprises a proximal port 9 connected with a lumen, the distal opening of which is located in the central vein (vena cava superior) 10. This lumen is used for injecting a bolus with a temperature different (usually lower) from the patient's blood temperature, thus introducing a travelling temperature deviation to the blood stream. As an alternative, a temperature deviation may be introduced by local heating or cooling in the central vein using a catheter equipped with heating (such as a heating coil) or cooling means (such as a Peltier-element), respectively. A thermodilution setup and a central venous catheter assembly equipped with means for local heating of central venous blood, which can be implemented correspondingly in a right heart catheter 1 assembly as well, are described in US 6,736,782.

Preferably, the proximal port 9 is equipped with an injection channel 11 for accomplishing a bolus injection as well-defined as possible in terms of time and duration of bolus injection as well as bolus temperature. For this, the injection channel 11 may comprise a pressure switch and a temperature sensor connected with the patient monitor 4 via a transducer 12. The injection channel may be configured as described in US 6,491,640.

Optionally, the central venous catheter 24 may be equipped with a distal pressure sensor for measuring blood pressure in the central vein 10. The sensor signal is transmitted to the patient monitor 4 via a proximal catheter port 5 and transducer 26.

The system further comprises an arterial catheter 17 comprising a temperature sensor for measuring the local blood temperature in an artery 18 and a pressure sensor for measuring the arterial blood pressure. Each of the sensors is connected with the patient monitor 4 via proximal catheter ports 19, 20 and transducers 21, 22, respectively. Though the arterial catheter 17 is placed in a femoral artery in the schematic view of fig. 1, catheter placement in other arteries such as axillary (brachial) artery or radial artery may also be suitable.

The temperature deviation introduced to the patient's 3 blood stream by bolus injection or local heating dilutes while travelling through the right heart 13, pulmonary circulation 14, left heart 15 and systemic circulation 16. This dilution is evaluated by applying known thermodilution algorithms using temperature over time measurements performed with the temperature sensor of the arterial catheter 17. The patient monitor 4 is adapted to perform this evaluation using an evaluation program stored in a memory of the patient monitor 4.

In particular, global enddiastolic volume GEDV is determined in the following manner.

$$GEDV = ITTV - PTV$$

wherein ITTV is the intrathoracic thermo volume and PTV is the pulmonary thermo volume. These parameters are determined as follows

$$ITTV = CO \cdot MTtTDa$$

$$PTV = CO \cdot DStTDa$$

wherein MTtTDa is the mean transit time and DStTDa is the downslope time (i.e. the time the blood temperature difference $\Delta TB(t)$ takes to drop by the factor 1/e where the dilution curve shows exponential decay) both determined from the dilution curve measured by the transpulmonary setup. CO is the cardiac output and may be determined either by pulse contour analysis using known algorithms (such as disclosed in US 6,315,735) based on the pressure-over-time signal measured with the pressure sensor of the arterial catheter 17, or it may be determined (using the temperature-over-time signal measured with the temperature sensor of the arterial catheter 17) by known thermodilution algorithms based on the Stewart-Hamilton equation

$$CO = \frac{V_L(T_B - T_L)K_1 K_2}{\int \Delta T_B(t)dt}$$

wherein $T_B$ is the initial blood temperature, $T_L$ is the temperature of the liquid bolus, which is used as thermal indicator, $V_L$ is the thermal indicator volume, $K_1$ and $K_2$ are constants to consider the specific measurement setup, and $\Delta TB(t)$ is the blood temperature as a function of time with respect to the baseline blood temperature $T_B$.

A parameter representing extravascular lung water EVLW can be determined as the difference between Intrathoracic Thermovolume ITTV and the Intrathoracic blood volume ITBV

$$EVLW = ITTV - ITBV$$

In the present embodiment, EVLW is normalised with a predicted (ideal) body weight PBW which is calculated from the patient's 3 body height:

$$EVLWi = EVLW / PBW$$

wherein EVLWi is the normalised value.

The memory of the patient monitor 4 has stored therein a program for selectively executing one of six algorithms for determining a predicted weight.

- The first algorithm is optimized for determining the predicted weight of a female adult with a height above a first threshold value of patient's height.
- The second algorithm is optimized for determining the predicted weight of a male adult with a height above a second threshold value of patient's height.
- The third algorithm is optimized for determining the predicted weight of a female pediatric patient with a height above a third threshold value of patient's height.

- The fourth algorithm is optimized for determining the predicted weight of a male pediatric patient with a height above a fourth threshold value of patient's height.
- The fifth algorithm is optimized for determining the predicted weight of a female pediatric patient with a height below the third threshold value of patient's height.
- The sixth algorithm is optimized for determining the predicted weight of a male pediatric patient with a height below the fourth threshold value of patient's height.

As an example, predicted weight algorithms may be based on available validated formulas derived from well founded empiric data from *The Acute Respiratory Distress Syndrome Network: "Ventilation With Lower Tidal Volumes As Compared With Traditional Tidal Volumes For Acute Lung Injury And The Acute Respiratory Distress Syndrome"* in The New England Journal of Medicine 342 (2000) 1301-1308:

- first algorithm: predicted weight [kg] = 50 + 0.91 · (height [cm] -130)
- second algorithm: predicted weight [kg] = 45.5 + 0.91 · (height [cm] - 130)

and from Traub, Scott L. and Johnson, Cary E.: "Comparison of methods of estimating creatine clearance in children" in American Journal of Hospital Pharmacy 37 (1980) 195-201:

- third algorithm: predicted weight [kg] = 39 + 0.89 (height [cm] -152.4)
- fourth algorithm: predicted weight [kg] = 42.2 + 0.89 · (height [cm] - 152.4)
- fifth algorithm: predicted weight [kg] =(height [cm] · 1.65) /1000
- sixth algorithm: predicted weight [kg] =(height [cm] · 1.65) /1000

In this case, the first and second threshold values amount to 152.4 cm and are thus identical with each other and will be referred to simply as "adult threshold value" hereinafter. Likewise, the third and fourth threshold values amount to 152.4 cm and are thus identical with each other and will be referred to simply as "pediatric threshold value" hereinafter. It is to be understood, however, that varying threshold values may be used if they become available. Further, fifth and sixth algorithms are identical with each other and will be referred to simply as "fifth algorithm" hereinafter. As above, it is to be understood, however, that differing algorithms may be used if they are available.

It can also be seen that the above formulas on which the first, second, third and fourth algorithms are based, all share the form

$$\text{predicted weight [kg]} = A + B \cdot (\text{height [cm]} - C)$$

Selecting the desired algorithm can thus be performed by simply assigning a certain value to the coefficient B or the constants A and C, respectively.

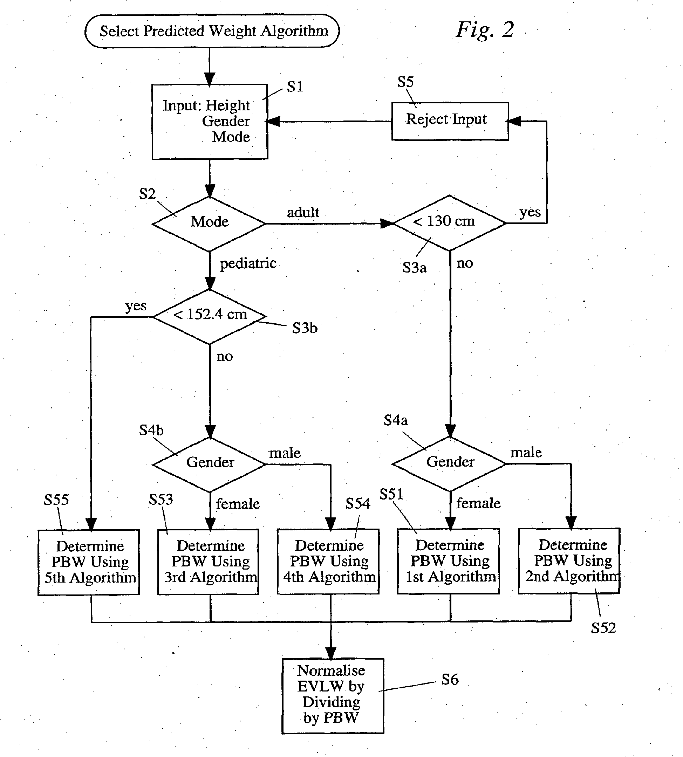

An algorithm selection procedure is schematically illustrated in fig. 2.

Before determining normalised values of extravascular lung water EVLW, the operator is prompted, e.g. by a respective dialog box displayed on display 23, to input height and gender of the patient 3 and whether the patient 3 is an adult (adult mode) or a pediatric patient (pediatric mode) - step S1 in figure 2. For this input, a keyboard 1 or any other suitable input means (such as a touch screen, a track ball or the like) may be used.

The patient monitor 4 then allocates the patient 3 to a category corresponding to one of the above algorithms, as illustrated in fig. 2, and determines the predicted weight ("predicted body weight" PBW) from the patient's 3 height using the respective algorithm (1st, 2nd, 3rd, 4th or 5th algorithm).

If it is determined (steps S2 and S3a) that adult mode is selected and the patient's 3 height is below the adult threshold value of - in the present example - 130 cm (or the first or second threshold value, respectively), and thus outside the range for which the first and second algorithms are validated, the selection of adult mode is rejected (step S5) and it is left to a physician to decide whether the patient 3 is to be treated as a pediatric patient.

It is to be understood, however, that algorithms validated for adults with a height below the adult threshold value (or the first or second threshold value, respectively) may be used instead of rejecting the selection of the adult mode, if such algorithms are available.

If it is determined (steps S2 and S3a) that adult mode is selected and the patient's 3 height is equla to or above the adult threshold value of - in the present example - 130 cm (or the first or second threshold value, respectively), and thus within the range for which the first and second algorithms are validated, the first algorithm is selected in case the gender is female and the second algorithm is selected in case the gender is male (step S4a)

If it is determined (steps S2 and S3b) that pediatric mode is selected and the patient's 3 height is equal to or above the pediatric threshold value of - in the present example - 152.4 cm (or the third or fourth threshold value, respectively), and thus within the range for which the third and fourth algorithms are validated, the third algorithm is selected in case the gender is female and the fourth algorithm is selected in case the gender is male (step S4b)

If it is determined (steps S2 and S3b) that pediatric mode is selected and the patient's 3 height is below the pediatric threshold value of - in the present example - 152.4 cm (or the third or fourth threshold value, respectively), and thus within the range for which the fifth algorithm is validated, the fifth algorithm is selected.

The predicted weight PBW is determined using the appropriate respective algorithm (step S51, S52, S53 S54, S55).

The extra vascular lung water determined as described above is then normalised by dividing by the predicted weight PBW (step S6). The normalised value EVLWi is displayed on the display 23 and/or otherwise output and may be stored in a memory or on a suitable external storage medium.

Further, global enddiastolic volume GEDV is normalised by dividing by a notional body surface area ("predicted body surface") PBSA to yield the normalised value GEDVi.

$$GEDVi = GEDV / PBSA$$

The predicted body surface area PBSA can be determined, for example, using the following formula, if PBW is below 15 kg

$$PBSA = (PBW^{0.5378} \cdot height\ [cm]^{0.3964}) \cdot 0.24265$$

and using the following formula, if PBW is equal to or above 15 kg

$$PBSA = (PBW^{0.425} \cdot height\ [cm]^{0.725}) \cdot 0.007184$$

wherein PBW is the predicted body weight determined as described above.

Generally, a predicted body surface area can be determined by using a weight dependent known formula for body surface area and substituting actual weight with predicted body weight. The various algorithms for determining predicted body weight can thus be considered subalgorithms of respective algorithms for determining body surface area.

The thus normalised value is displayed on the display 23 and/or otherwise output and may be stored in a memory or on a suitable external storage medium.

## Claims

1. A patient monitoring apparatus for determining a parameter of a patient (3), said patient monitoring apparatus comprising computing means adapted to

   - read in sensor data representing at least one physical quantity measured at a predetermined position of the patient's (3) cardiovascular system as a function of time, and to
   - determine said parameter representing said intrathoracic volume compartment using said sensor data,

   said patient monitoring apparatus being **characterized in that** said patient monitoring apparatus further comprises input means (1) for inputting patient (3) specific information, said patient (3) specific information including categorizing information and biometric information, and **in that** said computing means are further adapted

   - to allocate said patient (3) to a patient category depending on said categorizing information,
   - to select, depending on said patient category, an algorithm from a plurality of algorithms usable for normalizing said parameter representing said intrathoracic volume compartment using said biometric information, and
   - to normalize said parameter representing an intrathoracic volume compartment using said algorithm,

   wherein said intrathoracic volume compartment is extravascular lung water, intrathoracic blood volume, pulmonary blood volume or global enddiastolic volume,
   wherein said algorithms are algorithms for determining a notional weight of said patient (3) using said biometric

information, <u>wherein said biometric information includes said patient's (3) height,</u> and
said computing means are adapted to normalise said parameter representing said intrathoracic volume compartment with said notional weight.

2. A patient monitoring apparatus according to any of the preceding claims, wherein at least one of said physical quantities is a local blood temperature and said computing means are adapted to

- process information characterizing an initial local temperature change in the proximity of another predetermined position of the patient's (3) cardiovascular system, and
- to determine said parameter representing said intrathoracic volume compartment from said sensor data and said information characterizing said initial local temperature change using a thermodilution algorithm.

3. A patient monitoring apparatus according to claim 2, wherein said computing means are adapted to process information characterizing the initial local temperature change in such a manner that said computing means control effecting the initial local temperature change in accordance with said information.

4. A patient monitoring apparatus according to claim 2, wherein said computing means are adapted to read in the information characterizing the initial local temperature change for processing.

5. A patient monitoring apparatus according to any of claims 2-4, wherein said patient monitoring apparatus further comprises

- temperature influencing means (11) for effecting the initial local temperature change in the proximity of said other position of the patient's (3) cardiovascular system, thus introducing a travelling temperature deviation to the patient's (3) blood stream, and
- temperature sensing means (21) connected to said computing means for measuring said local blood temperature and providing said sensor data.

6. A patient monitoring apparatus for determining a parameter of a patient (3), said patient monitoring apparatus comprising computing means adapted to

- read in sensor data representing at least one physical quantity measured at a predetermined position of the patient's (3) cardiovascular system as a function of time, and to
- determine said parameter representing said intrathoracic volume compartment using said sensor data,

said patient monitoring apparatus being **characterized in that** said patient monitoring apparatus further comprises input means (1) for inputting patient (3) specific information, said patient (3) specific information including categorizing information and biometric information, and **in that** said computing means are further adapted

- to allocate said patient (3) to a patient category depending on said categorizing information,
- to select, depending on said patient category, an algorithm from a plurality of algorithms usable for normalizing said parameter representing said intrathoracic volume compartment using said biometric information, and
- to normalize said parameter representing an intrathoracic volume compartment using said algorithm,

wherein said intrathoracic volume compartment is extravascular lung water, intrathoracic blood volume, pulmonary blood volume or global enddiastolic volume, and
wherein said algorithms are algorithms for determining a notional body surface area of said patient (3) using said biometric information, and said computing means are adapted to normalise said parameter representing said intrathoracic volume compartment with said notional body surface area, and
wherein said algorithms for determining a notional body surface area of said patient (3) comprise subalgorithms for determining a notional weight of said patient (3) using said biometric information, <u>wherein said biometric information includes said patient's (3) height</u>.

7. A patient monitoring apparatus according to any of the preceding claims, wherein at least part of said biometric information is identical with at least part of said categorizing information.

8. A patient monitoring apparatus according to any of the preceding claims, wherein said categorizing information includes said patient's (3) gender.

**9.** A patient monitoring apparatus according to any of the preceding claims, wherein said categorizing information includes mode information, said mode information indicating whether said patient (3) is to be treated as an adult patient or as a pediatric, patient, and said plurality of algorithms include at least one pediatric mode algorithm and at least one adult mode algorithm.

**10.** A patient monitoring apparatus according to claim 9, which is adapted to reject said mode information, if said mode information indicates that said patient (3) is to be treated as an adult patient and said biometric information includes a biometric value outside a predetermined range, for which said at least one adult mode algorithm is valid, or if said mode information indicates that said patient (3) is to be treated as a pediatric patient and said biometric information includes a biometric value outside a predetermined range, for which said at least one pediatric mode algorithm is valid.

**11.** A method of determining a parameter representing an intrathoracic volume compartment of a patient (3), wherein

- sensor data is read in representing at least one physical quantity measured at a predetermined position of the patient's (3) cardiovascular system as a function of time, and
- said parameter representing said intrathoracic volume compartment is determined using said sensor data,

said method being **characterised in that** it includes

- acquiring patient specific information (S1), said patient specific information including categorizing information and biometric information,
- allocating (S2, S3a, S3b, S4a, S4b) said patient (3) to a patient category depending on said categorizing information,
- selecting, depending on said patient category, an algorithm from a plurality of algorithms usable for normalising said parameter representing said intrathoracic volume compartment using said biometric information, and
- normalising (S51, S52, S53, S54, S55, S6) said parameter representing said intrathoracic volume compartment using said algorithm,

wherein said intrathoracic volume compartment is extravascular lung water, intrathoracic blood volume, pulmonary blood volume or enddiastolic volume, and
wherein said algorithms are algorithms for determining a notional weight of said patient (3) using said biometric information, wherein said biometric information includes said patient's (3) height, and
said parameter representing said intrathoracic volume compartment is normalised with said notional weight.

**12.** A method according to claim 11, wherein at least one of said physical quantities is a local blood temperature and said parameter representing said intrathoracic volume compartment is determined from said sensor data and information characterizing an initial local temperature change in the proximity of another predetermined position of the patient's (3) cardiovascular system using a thermodilution algorithm.

**13.** A method of determining a parameter representing an intrathoracic volume compartment of a patient (3), wherein

- sensor data is read in representing at least one physical quantity measured at a predetermined position of the patient's (3) cardiovascular system as a function of time, and
- said parameter representing said intrathoracic volume compartment is determined using said sensor data,

said method being **characterised in that** it includes

- acquiring patient specific information (S1), said patient specific information including categorizing information and biometric information,
- allocating (S2, S3a, S3b, S4a, S4b) said patient (3) to a patient category depending on said categorizing information,
- selecting, depending on said patient category, an algorithm from a plurality of algorithms usable for normalising said parameter representing said intrathoracic volume compartment using said biometric information, and
- normalising (S51, S52, S53, S54, S55, S6) said parameter representing said intrathoracic volume compartment using said algorithm,

wherein said intrathoracic volume compartment is extravascular lung water, intrathoracic blood volume, pulmonary blood volume or enddiastolic volume, and

wherein said algorithms are algorithms for determining a notional body surface area of said patient (3) using said biometric information, and said parameter representing said intrathoracic volume compartment is normalised with said notional body surface area, and wherein said algorithms for determining a notional body surface area of said patient (3) comprise subalgorithms for determining a notional weight of said patient (3) using said biometric information, wherein said biometric information includes said patient's (3) height.

**14.** A method according to any of claims 11-13, wherein at least part of said biometric information is identical with at least part of said categorizing information.

**15.** A method according to any of claims 11-14, wherein said categorizing information includes said patient's (3) gender.

**16.** A method according to any of claims 11-15, wherein said categorizing information includes mode information, said mode information indicating whether said patient (3) is to be treated as an adult patient or as a pediatric patient, and said plurality of algorithms include at least one pediatric mode algorithm and at least one adult mode algorithm.

**17.** A method according to claim 16, wherein said mode information is rejected (SS), if said mode information indicates that said patient (3) is to be treated as an adult patient and said biometric information includes a biometric value outside a predetermined range, for which said at least one adult mode algorithm is valid, or if said mode information indicates that said patient (3) is to be treated as a pediatric patient and said biometric information includes a biometric value outside a predetermined range, for which said at least one pediatric mode algorithm is valid.


**Patentansprüche**

**1.** Patientenüberwachungs-Vorrichtung zum Bestimmen eines Parameters eines Patienten (3), wobei die Patientenüberwachungs-Vorrichtung Berechnungsmittel umfasst, welche ausgelegt sind

- zum Einlesen von Sensordaten, welche wenigstens eine an einer vorbestimmten Position des kardiovaskulären Systems des Patienten (3) als Funktion der Zeit gemessene physikalische Größe repräsentieren, und
- zum Bestimmen des Parameters, welcher das intrathorakale Volumenkompartiment repräsentiert, mittels der Sensordaten,

wobei die Patientenüberwachungs-Vorrichtung **dadurch gekennzeichnet ist,**
**dass** die Patientenüberwachungs-Vorrichtung weiterhin Eingabemittel (1) zur Eingabe Patienten(3)-spezifischer Information umfasst, wobei die Patienten(3)-spezifische Information kategorisierende Information und biometrische Information einschließt, und
**dass** die Berechnungsmittel weiterhin dazu ausgelegt sind,

- den Patienten (3) in Abhängigkeit von der kategorisierenden Information einer Patientenkategorie zuzuordnen,
- in Abhängigkeit von der Patientenkategorie unter Verwendung der biometrischen Information einen Algorithmus aus mehreren Algorithmen auszuwählen, welche für die Normalisierung des das intrathorakale Volumenkompartiment repräsentierenden Parameters anwendbar sind, und
- den ein intrathorakales Volumenkompartiment repräsentierenden Parameter mittels des Algorithmus zu normalisieren,

wobei das intrathorakale Volumenkompartiment extravasales Lungenwasser, intrathorakales Blutvolumen, pulmonales Blutvolumen oder globales enddiastolisches Volumen ist,
wobei die Algorithmen Algorithmen zum Bestimmen des fiktiven Gewichts des Patienten (3) mittels der biometrischen Information sind, wobei die biometrische Information die Größe des Patienten (3) einschließt,
und die Berechnungsmittel dazu ausgelegt sind, den das intrathorakale Volumenkompartiment repräsentierenden Parameter mittels des fiktiven Gewichts zu normalisieren.

**2.** Patientenüberwachungs-Vorrichtung gemäß Anspruch 1, wobei wenigstens eine der physikalischen Größen eine lokale Bluttemperatur ist und die Berechnungsmittel ausgelegt sind

- zum Verarbeiten von Information, welche eine initiale lokale Temperaturänderung in der Nähe einer anderen vorbestimmten Position des kardiovaskulären Systems des Patienten (3) kennzeichnet, und
- zum Bestimmen des ein intrathorakales Volumenkompartiment repräsentierenden Parameters aus den Sensordaten und der die initiale lokale Temperaturänderung kennzeichnenden Information mittels eines Thermodilutions-Algorithmus.

3. Patientenüberwachungs-Vorrichtung gemäß Anspruch 2, wobei die Berechnungsmittel zum Verarbeiten von Information, welche die initiale lokale Temperaturänderung kennzeichnen, derart ausgelegt sind, dass die Berechnungsmittel in Übereinstimmung mit dieser Information das Herbeiführen der initialen lokalen Temperaturänderung steuern.

4. Patientenüberwachungs-Vorrichtung gemäß Anspruch 2, wobei die Berechnungsmittel zum Einlesen der die initiale lokale Temperaturänderung kennzeichnenden Information zum Verarbeiten ausgelegt sind.

5. Patientenüberwachungs-Vorrichtung gemäß einem der Ansprüche 2 bis 4, wobei die Patientenüberwachungs-Vorrichtung weiterhin umfasst

   - Temperatur-Beeinflussungsmittel (11) zum Herbeiführen der initialen, lokalen Temperaturänderung in der Nähe der anderen Position des kardiovaskulären Systems des Patienten (3), wodurch eine wandernde Temperaturabweichung im Blutstrom des Patienten (3) hervorgerufen wird, und
   - mit den Berechnungsmitteln verbundene Temperatur-Messmittel (21), welche die lokale Bluttemperatur messen und die Sensordaten bereitstellen.

6. Patientenüberwachungs-Vorrichtung zum Bestimmen eines Parameters eines Patienten (3), wobei die Patientenüberwachungs-Vorrichtung Berechnungsmittel umfasst, welche ausgelegt sind

   - zum Einlesen von Sensordaten, welche wenigstens eine an einer vorbestimmten Position des kardiovaskulären Systems des Patienten (3) als Funktion der Zeit gemessene physikalische Größe repräsentieren, und
   - zum Bestimmen des Parameters, welcher das intrathorakale Volumenkompartiment repräsentiert, mittels der Sensordaten,

   wobei die Patientenüberwachungs-Vorrichtung **dadurch gekennzeichnet ist,**
   **dass** die Patientenüberwachungs-Vorrichtung weiterhin Eingabemittel (1) zur Eingabe Patienten(3)-spezifischer Information umfasst, wobei die Patienten (3)-spezifische Information kategorisierende Information und biometrische Information einschließt, und dass die Berechnungsmittel weiterhin dazu ausgelegt sind,

   - den Patienten (3) in Abhängigkeit von der kategorisierenden Information einer Patientenkategorie zuzuordnen,
   - in Abhängigkeit von der Patientenkategorie unter Verwendung der biometrischen Information einen Algorithmus aus mehreren Algorithmen auszuwählen, welche für die Normalisierung des das intrathorakale Volumenkompartiment repräsentierenden Parameters anwendbar sind, und
   - den ein intrathorakales Volumenkompartiment repräsentierenden Parameter mittels des Algorithmus zu normalisieren,

   wobei das intrathorakale Volumenkompartiment extravales Lungenwasser, intrathorakales Blutvolumen, pulmonales Blutvolumen oder globales enddiastolisches Volumen ist,
   wobei die Algorithmen Algorithmen zum Bestimmen der fiktiven Körperoberfläche des Patienten (3) mittels der biometrischen Information sind, und die Berechnungsmittel dazu ausgelegt sind, den das intrathorakale Volumenkompartiment repräsentierenden Parameter mittels der fiktiven Körperoberfläche zu normalisieren, und
   wobei die Algorithmen zur Bestimmung der fiktiven Körperoberfläche des Patienten (3) Subalgorithmen zum Bestimmen des fiktiven Gewichts des Patienten mittels der biometrischen Information umfassen, wobei die biometrischen Information die Größe des Patienten (3) einschließt.

7. Patientenüberwachungs-Vorrichtung gemäß einem der vorangehenden Ansprüche, wobei wenigstens ein Teil der biometrischen Information identisch mit wenigstens einem Teil der kategorisierenden Information ist.

8. Patientenüberwachungs-Vorrichtung gemäß einem der vorangehenden Ansprüche, wobei die kategorisierende Information das Geschlecht des Patienten (3) einschließt.

9. Patientenüberwachungs-Vorrichtung gemäß einem vorangehenden Ansprüche, wobei die kategorisierende Information Modus-Information einschließt, wobei die Modus-Information anzeigt, ob der Patient (3) als erwachsener Patient oder als pädiatrischer Patient zu behandeln ist, und wobei die mehreren Algorithmen wenigstens einen Algorithmus für den pädiatrischen Modus und wenigstens einen Algorithmus für den Erwachsenen-Modus einschließen.

10. Patientenüberwachungs-Vorrichtung gemäß Anspruch 9, welcher dazu eingerichtet ist, die Modus-Information zurückzuweisen, wenn die Modus-Information anzeigt, dass der Patient (3) als ein erwachsener Patient zu bearbeiten ist und die biometrische Information einen biometrischen Wert außerhalb eines vorher bestimmten Bereichs einschließt, für den der wenigstens eine Erwachsenen-Modus Algorithmus gültig ist, oder wenn die Modus-Information anzeigt, dass der Patient (3) als ein pädiatrischer Patient zu behandeln ist und die biometrische Information einen biometrischen Wert außerhalb eines vorher bestimmten Bereichs einschließen, für den wenigstens ein Algorithmus des pädiatrischen Modus gültig ist.

11. Verfahren zur Bestimmung eines Parameters, welcher ein intrathorakales Volumenkompartiment eines Patienten (3) repräsentiert, wobei

- Sensordaten eingelesen werden, welche wenigstens eine an einer vorbestimmten Position des kardiovaskulären Systems des Patienten (3) als Funktion der Zeit gemessene physikalische Größe repräsentieren, und
- der Parameter, welcher das intrathorakale Volumenkompartiment repräsentiert, mittels der Sensordaten bestimmt wird,

wobei das Verfahren **dadurch gekennzeichnet ist, dass** es beinhaltet

- Erfassen patientenspezifischer Information (S1), wobei die patientenspezifische Information kategorisierende Information und biometrische Information umfasst,
- Zuordnen (S2, S3a, S3b, S4a, S4b) des Patienten (3) zu einer Patientenkategorie in Abhängigkeit von der kategorisierenden Information,
- Auswählen, in Abhängigkeit von der Patientenkategorie, eines Algorithmus aus mehreren Algorithmen, welche für die Normalisierung des das intrathorakale Volumenkompartiment repräsentierenden Parameters anwendbar sind, unter Verwendung der biometrischen Information,
- Normalisieren (S51, S52, S53, S54, S55, S6) des das intrathorakale Volumenkompartiment repräsentierenden Parameters mittels des Algorithmus,

wobei das intrathorakale Volumenkompartiment extravasles Lungenwasser, intrathorakales Blutvolumen, pulmonales Blutvolumen oder globales enddiastolisches Volumen ist, und wobei die Algorithmen Algorithmen zum Bestimmen des fiktiven Gewichts des Patienten (3) mittels der biometrischen Information sind, wobei die biometrische Information die Größe des Patienten (3) einschließen,
und wobei der das intrathorakale Volumenkompartiment repräsentierende Parameter mittels des fiktiven Gewichts normalisiert wird.

12. Verfahren gemäß Anspruch 11, wobei wenigstens eine der physikalischen Größen eine lokale Bluttemperatur ist und der das intrathorakale Volumenkompartiment repräsentierende Parameter mittels eines Thermodilutions-Algorithmus bestimmt wird aus den Sensordaten und aus Information, welche eine initiale lokale Temperaturänderung in der Nähe einer anderen vorbestimmten Position des kardiovaskulären Systems des Patienten (3) kennzeichnet.

13. Verfahren zur Bestimmung eines Parameters, welcher ein intrathorakales Volumenkompartiment eines Patienten (3) repräsentiert, wobei

- Sensordaten eingelesen werden, welche wenigstens eine an einer vorbestimmten Position des kardiovaskulären Systems des Patienten (3) als Funktion der Zeit gemessene physikalische Größe repräsentieren, und
- der Parameter, welcher das intrathorakale Volumenkompartiment repräsentiert, mittels der Sensordaten bestimmt wird,

wobei das Verfahren **dadurch gekennzeichnet ist, dass** es beinhaltet

- Erfassen Patienten-spezifischer Information (S1), wobei die patientenspezifische Information kategorisierende Information und biometrische Information umfasst,
- Zuordnen (S2, S3a, S3b, S4a, S4b) des Patienten (3) zu einer Patientenkategorie in Abhängigkeit von der kategorisierenden Information,
- Auswählen, in Abhängigkeit von der Patientenkategorie, eines Algorithmus aus mehreren Algorithmen, welche für die Normalisierung des ein intrathorakales Volumenkompartiment repräsentierenden Parameters anwendbar sind, unter Verwendung der biometrischen Information,
- Normalisieren (S51, S52, S53, S54, S55, S6) des das intrathorakale Volumenkompartiment repräsentierenden Parameters mittels des Algorithmus,

wobei das intrathorakale Volumenkompartiment extravasales Lungenwasser, intrathorakales Blutvolumen, pulmonales Blutvolumen oder globales enddiastolisches Volumen ist, und wobei die Algorithmen Algorithmen zum Bestimmen der fiktiven Körperoberfläche des Patienten (3) mittels der biometrischen Information sind, und wobei der das intrathorakale Volumenkompartiment repräsentierende Parameter mittels der fiktiven Körperoberfläche normalisiert wird, und wobei die Algorithmen zur Bestimmung der fiktiven Körperoberfläche des Patienten (3) Subalgorithmen zum Bestimmen des fiktiven Gewichts des Patienten mittels der biometrischen Information umfassen, wobei die biometrische Information die Größe des Patienten (3) einschließt.

14. Verfahren gemäß einem der Ansprüche 11 bis 13, wobei wenigstens ein Teil der biometrischen Information identisch mit wenigstens einem Teil der kategorisierenden Information ist.

15. Verfahren gemäß einem der Ansprüche 11 bis 14, wobei die kategorisierende Information das Geschlecht des Patienten (3) einschließt.

16. Verfahren gemäß einem der Ansprüche 11 bis 15, wobei die kategorisierende Information Modus-Information einschließt, wobei die Modus- Information anzeigt, ob der Patient (3) als erwachsener Patient oder als pädiatrischer Patient zu behandeln ist, und wobei die mehreren Algorithmen wenigstens einen Algorithmus für den pädiatrischen Modus und wenigstens einen Algorithmus für den Erwachsenen-Modus einschließen.

17. Verfahren gemäß Anspruch 16, wobei die Modus-Information zurückgewiesen wird (SS), wenn die Modus-Information anzeigt, dass der Patient (3) als ein erwachsener Patient zu behandeln ist und die biometrische Information einen biometrischen Wert außerhalb eines vorher bestimmten Bereichs einschließt, für den wenigstens ein Erwachsenen-Modus Algorithmus gültig ist, oder wenn die Modus-Information anzeigt, dass der Patient (3) als ein pädiatrischer Patient zu behandeln ist und die biometrische Information einen biometrischen Wert außerhalb eines vorher bestimmten Bereichs einschließt, für den wenigstens ein Algorithmus des pädiatrischen Modus gültig ist.

## Revendications

1. Appareil de surveillance d'un patient, pour déterminer un paramètre d'un patient (3), ledit appareil de surveillance d'un patient comprenant des moyens de calcul adaptés pour

- lire des données de capteur représentant au moins une quantité physique mesurée sur une position prédéterminée du système cardiovasculaire du patient (3) en fonction du temps, et pour
- déterminer ledit paramètre représentant ledit compartiment du volume intrathoracique en utilisant lesdites données de capteur,

ledit appareil de surveillance d'un patient étant **caractérisé en ce que** ledit appareil de surveillance d'un patient comprend en outre des moyens d'entrée (1) pour entrer des informations spécifiques d'un patient (3), lesdites informations spécifiques d'un patient (3) comprenant des informations de catégorisation et des informations biométriques, et **en ce que** lesdits moyens de calcul sont en outre adaptés

- pour affecter ledit patient (3) à une catégorie de patient en fonction desdites informations de catégorisation,
- pour choisir, en fonction de ladite catégorie de patient, un algorithme parmi une pluralité d'algorithmes utilisables pour la normalisation dudit paramètre représentant ledit compartiment du volume intrathoracique, utilisant ladite information biométrique, et
- pour normaliser ledit paramètre représentant un compartiment du volume intrathoracique en utilisant ledit algorithme,

dans lequel ledit compartiment du volume intrathoracique est de l'eau pulmonaire extravasculaire, un volume sanguin intrathoracique, un volume sanguin pulmonaire ou un volume de fin de diastole global
dans lequel lesdits algorithmes sont des algorithmes de détermination d'un poids notionnel dudit patient (3) utilisant lesdites informations biométriques, dans lequel lesdites informations biométriques comprennent la taille dudit patient (3), et

lesdits moyens de calcul sont adaptés pour normaliser ledit paramètre représentant ledit compartiment du volume intrathoracique avec ledit poids notionnel.

2. Appareil de surveillance d'un patient selon l'une quelconque des revendications précédentes, dans lequel au moins l'une desdites quantités physiques est une température sanguine locale et lesdits moyens de calcul sont adaptés pour

- traiter des informations caractérisant un changement de température locale initiale à proximité d'une autre position prédéterminée du système cardiovasculaire du patient (3), et
- déterminer ledit paramètre représentant ledit compartiment du volume intrathoracique à partir desdites données de capteur et desdites informations caractérisant ledit changement de température locale initiale en utilisant un algorithme de thermodilution.

3. Appareil de surveillance d'un patient selon la revendication 2, dans lequel lesdits moyens de calcul sont adaptés pour traiter des informations caractérisant le changement de température locale initiale de telle sorte que lesdits moyens de calcul contrôlent l'affectation du changement de température locale initiale selon lesdites informations.

4. Appareil de surveillance d'un patient selon la revendication 2, dans lequel lesdits moyens de calcul sont adaptés pour lire les informations caractérisant le changement de température locale initiale pour le traitement.

5. Appareil de surveillance d'un patient selon l'une quelconque des revendications 2 à 4, dans lequel ledit appareil de surveillance d'un patient comprend en outre

- des moyens (11) influant sur la température pour affecter le changement de température locale initiale à proximité de ladite autre position du système cardiovasculaire du patient (3), introduisant ainsi un écart de température mobile dans la circulation sanguine du patient (3), et
- des moyens de capture de la température (21) reliés auxdits moyens de calcul pour mesurer ladite température sanguine locale et fournir lesdites données de capteur.

6. Appareil de surveillance d'un patient pour déterminer un paramètre d'un patient (3), ledit appareil de surveillance d'un patient comprenant des moyens de calcul adaptés pour

- lire des données de capteur représentant au moins une quantité physique mesurée sur une position prédéterminée du système cardiovasculaire du patient (3) en fonction du temps, et pour
- déterminer ledit paramètre représentant ledit compartiment du volume intrathoracique en utilisant lesdites données de capteur,

ledit appareil de surveillance d'un patient étant **caractérisé en ce que** ledit appareil de surveillance d'un patient comprend en outre des moyens d'entrée (1) pour entrer des informations spécifiques d'un patient (3), lesdites informations spécifiques d'un patient (3) comprenant des informations de catégorisation et des informations biométriques, et **en ce que** lesdits moyens de calcul sont en outre adaptés

- pour affecter ledit patient (3) à une catégorie de patient en fonction desdites informations de catégorisation,
- pour choisir, en fonction de ladite catégorie de patient, un algorithme parmi une pluralité d'algorithmes utilisables pour la normalisation dudit paramètre représentant ledit compartiment du volume intrathoracique utilisant ladite information biométrique, et
- pour normaliser ledit paramètre représentant un compartiment du volume intrathoracique en utilisant ledit algorithme,

dans lequel ledit compartiment du volume intrathoracique est de l'eau pulmonaire extravasculaire, un volume sanguin intrathoracique, un volume sanguin pulmonaire ou un volume de fin de diastole global et
dans lequel lesdits algorithmes sont des algorithmes de détermination d'une surface corporelle notionnelle dudit patient (3) utilisant lesdites informations biométriques et lesdits moyens de calcul sont adaptés pour normaliser ledit paramètre représentant ledit compartiment du volume intrathoracique avec ladite surface corporelle notionnelle, et
dans lequel lesdits algorithmes de détermination d'une surface corporelle notionnelle dudit patient (3) comprennent des sub-algorithmes de détermination d'un poids notionnel dudit patient (3) utilisant lesdites informations biométriques, dans lequel lesdites informations biométriques comprennent la taille dudit patient (3).

7. Appareil de surveillance d'un patient selon l'une quelconque des revendications précédentes, dans lequel au moins une partie desdites informations biométriques est identique à au moins une partie desdites informations de catégorisation.

8. Appareil de surveillance d'un patient selon l'une quelconque des revendications précédentes, dans lequel lesdites informations de catégorisation comprennent le sexe dudit patient (3).

9. Appareil de surveillance d'un patient selon l'une quelconque des revendications précédentes, dans lequel lesdites informations de catégorisation comprennent des informations de mode, lesdites informations de mode indiquant si ledit patient (3) doit être traité en tant que patient adulte ou en tant que patient pédiatrique, et ladite pluralité d'algorithmes comprend au moins un algorithme de mode pédiatrique et au moins un algorithme de mode adulte.

10. Appareil de surveillance d'un patient selon la revendication 9, qui est adapté pour rejeter lesdites informations de mode si lesdites informations de mode indiquent que ledit patient (3) doit être traité en tant que patient adulte et lesdites informations biométriques comprennent une valeur biométrique en dehors d'une plage prédéterminée, pour laquelle ledit au moins un algorithme de mode adulte est valide, ou si lesdites informations de mode indiquent que ledit patient (3) doit être traité en tant que patient pédiatrique et lesdites informations biométriques comprennent une valeur biométrique en dehors d'une plage prédéterminée, pour laquelle ledit au moins un algorithme de mode pédiatrique est valide.

11. Procédé de détermination d'un paramètre représentant un compartiment du volume intrathoracique d'un patient (3), dans lequel

   - des données de capteur sont lues, représentant au moins une quantité physique mesurée sur une position prédéterminée du système cardiovasculaire du patient (3) en fonction du temps, et
   - ledit paramètre représentant ledit compartiment du volume intrathoracique est déterminé en utilisant lesdites données de capteur,

   ledit procédé étant **caractérisé en ce qu'**il comprend

   - l'acquisition d'informations (S1) spécifiques d'un patient, lesdites informations spécifiques d'un patient comprenant des informations de catégorisation et des informations biométriques,
   - l'affectation (S2, S3a, S3b, S4a, S4b) dudit patient (3) à une catégorie de patient en fonction desdites informations de catégorisation,
   - la sélection, en fonction de ladite catégorie de patient, d'un algorithme parmi une pluralité d'algorithmes utilisables pour la normalisation dudit paramètre représentant ledit compartiment du volume intrathoracique utilisant lesdites informations biométriques, et
   - la normalisation (S51, S52, S53, S54, S55, S6) dudit paramètre représentant ledit compartiment du volume intrathoracique en utilisant ledit algorithme,

   dans lequel ledit compartiment du volume intrathoracique est de l'eau pulmonaire extravasculaire, un volume sanguin intrathoracique, un volume sanguin pulmonaire ou un volume de fin de diastole, et
   dans lequel lesdits algorithmes sont des algorithmes de détermination d'un poids notionnel dudit patient (3) utilisant lesdites informations biométriques, dans lequel lesdites informations biométriques comprennent la taille dudit patient (3), et
   ledit paramètre représentant ledit compartiment du volume intrathoracique est normalisé avec ledit poids notionnel.

12. Procédé selon la revendication 11, dans lequel au moins l'une desdites quantités physiques est une température sanguine locale et ledit paramètre représentant ledit compartiment du volume intrathoracique est déterminé à partir desdites données de capteurs et d'informations caractérisant un changement de température locale initiale à proximité d'une autre position prédéterminée du système cardiovasculaire du patient (3) en utilisant un algorithme de thermodiluation.

13. Procédé de détermination d'un paramètre représentant un compartiment du volume intrathoracique d'un patient (3), dans lequel

   - des données de capteur sont lues, représentant au moins une quantité physique mesurée sur une position prédéterminée du système cardiovasculaire du patient (3) en fonction du temps, et
   - ledit paramètre représentant ledit compartiment du volume intrathoracique est déterminé en utilisant lesdites données de capteur,

ledit procédé étant **caractérisé en ce qu'**il comprend

- l'acquisition d'informations (S1) spécifiques d'un patient, lesdites informations spécifiques d'un patient comprenant des informations de catégorisation et des informations biométriques,
- l'affectation (S2, S3a, S3b, S4a, S4b) dudit patient (3) à une catégorie de patient en fonction desdites informations de catégorisation,
- la sélection, en fonction de ladite catégorie de patient, d'un algorithme parmi une pluralité d'algorithmes utilisables pour la normalisation dudit paramètre représentant ledit compartiment du volume intrathoracique utilisant lesdites informations biométriques, et
- la normalisation (S51, S52, S53, S54, S55, S6) dudit paramètre représentant ledit compartiment du volume intrathoracique en utilisant ledit algorithme,

dans lequel ledit compartiment du volume intrathoracique est de l'eau pulmonaire extravasculaire, un volume sanguin intrathoracique, un volume sanguin pulmonaire ou un volume de fin de diastole, et
dans lequel lesdits algorithmes sont des algorithmes de détermination d'une surface corporelle notionnelle dudit patient (3) utilisant lesdites informations biométriques, et ledit paramètre représentant ledit compartiment du volume intrathoracique est normalisé avec ladite surface corporelle notionnelle et dans lequel lesdits algorithmes de détermination d'une surface corporelle notionnelle dudit patient (3) comprennent des sub-algorithmes de détermination d'un poids notionnel dudit patient (3) utilisant lesdites informations biométriques, dans lequel lesdites informations biométriques comprennent la taille dudit patient (3).

**14.** Procédé selon l'une quelconque des revendications 11 à 13, dans lequel au moins une partie desdites informations biométriques est identique à au moins une partie desdites informations de catégorisation.

**15.** Procédé selon l'une quelconque des revendications 11 à 14, dans lequel lesdites informations de catégorisation comprennent le sexe dudit patient (3).

**16.** Procédé selon l'une quelconque des revendications 11 à 15, dans lequel lesdites informations de catégorisation comprennent des informations de mode, lesdites informations de mode indiquant si ledit patient (3) doit être traité en tant que patient adulte ou en tant que patient pédiatrique, et ladite pluralité d'algorithmes comprend au moins un algorithme de mode pédiatrique et au moins un algorithme de mode adulte.

**17.** Procédé selon la revendication 16, dans lequel lesdites informations de mode sont rejetées (SS) si lesdites informations de mode indiquent que ledit patient (3) doit être traité en tant que patient adulte et lesdites informations biométriques comprennent une valeur biométrique en dehors d'une plage prédéterminée, pour laquelle ledit au moins un algorithme de mode adulte est valide, ou si lesdites informations de mode indiquent que ledit patient (3) doit être traité en tant que patient pédiatrique et lesdites informations biométriques comprennent une valeur biométrique en dehors d'une plage prédéterminée, pour laquelle ledit au moins un algorithme de mode pédiatrique est valide.

*Fig. 1*

*Fig. 2*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5526817 A
- US 6394961 B
- US 6537230 B
- US 6736782 B
- US 5383468 A
- WO 0612713591526 A
- US 6491640 B
- US 6315735 B

**Non-patent literature cited in the description**

- **SAKKA S. G.** Assessment of cardiac preload and extravascular lung water by single transpulmonary thermodilution. *Intensive Care Medicine,* February 2000, vol. 26 (2), ISSN 0342 - 4624, 180-187
- **EDWARD H. et al.** Body surface area prediction in normal-weight and obese patients. *American Journal of Physiology,* September 2001, vol. 281 (3), ISSN 0002 - 9513, E586-E591
- **JOHN D.** A linear Equation for estimating the body surface area in infants and children. *The Internet Journal of Anesthesiology,* 1998, vol. 2 (2), ISSN 1092 - 406X, 1-4
- Ventilation With Lower Tidal Volumes As Compared With Traditional Tidal Volumes For Acute Lung Injury And The Acute Respiratory Distress Syndrome. *The New England Journal of Medicine,* 2000, vol. 342, 1301-1308
- **TRAUB, SCOTT L. ; JOHNSON, CARY E.** Comparison of methods of estimating creatine clearance in children. *American Journal of Hospital Pharmacy,* 1980, vol. 37, 195-201